# EUROPEAN PATENT APPLICATION

(11) **EP 4 115 892 A1**
(43) Date of publication of application: **11.01.2023**
(21) Application number: 21382616.7
(22) Date of filing: 07.07.2021
(51) Int. Cl.: A61K 35/17, C12N 5/0783, A61K 39/00, C07K 14/725, C07K 14/52, A61P 35/00

(54) **IN VITRO METHOD FOR IMPROVING THE PRODUCTION OF EXOSOMES FROM CAR-T CELLS**

(71) Applicant: Fundación Pública Andaluza Progreso Y Salud, 41092 Sevilla (ES); Universidad de Granada, 18071 Granada (ES)
(72) Inventor: BENABDELLAH EL KHLANJI, Karim, 18016 Granada (ES); MARCHAL CORRALES, Juan Antonio, 18016 Granada (ES); MARTIN MOLINA, Francisco, 18016 Granada (ES); GARCÍA ORTEGA, M. Belén, 18100 Granada (ES); PAVLOVIC PAVLOVIC, Kristina, 18016 Granada (ES); GALINDO MORENO, Pablo, 18072 Granada (ES); AHEGET, Houssam, 18100 Granada (ES)
(74) Representative: Hoffmann Eitle

(57) **Abstract**

The present invention refers to the medical field. Particularly, it refers to a method for improving the production of exosomes (EXO) from CAR-T cells by modifying the expression of specific genes. The invention also refers to CAR-T cells, or EXO derived thereof, wherein the expression of specific genes have been modified, and to pharmaceutical compositions comprising said CART-cells or EXO derived thereof.

## Description

### FIELD OF THE INVENTION

The present invention refers to the medical field. Particularly, it refers to a method for improving the production of exosomes (EXO) from CAR-T cells by modifying the expression of specific genes. The invention also refers to CAR-T cells, or EXO derived thereof, wherein the expression of specific genes have been modified, and to pharmaceutical compositions comprising said CART-cells or EXO derived thereof.

### STATE OF THE ART

Between 65% and 80% of patients with leukemias or lymphomas respond to polychemotherapy protocols with which they are potentially curable. However, refractory patients or those who relapse after a first remission have a poor prognosis, with a probability of survival that does not exceed 15-20%. Since the beginning of the 21^{st} century, adoptive antitumor immunotherapy has been introduced into the cancer therapeutic arsenal, first with simple monoclonal antibodies (mAb) (for example rituximab), followed by the use of toxin-conjugated monoclonal antibodies (such as brentuximab vedotin), bispecific monoclonal antibodies (blinatumumab), and already in the second decade of the 21^{st} century, cellular immunotherapy. The inclusion of immunochemotherapy protocols in the routine treatment of these patients has changed the prognosis of hematological tumors, particularly of the B lineage. However, the best results in relapsed or refractory patients have come from the hand of cell-gene therapy with the generation of T lymphocytes expressing chimeric antigen receptors (Chimeric Antigen Receptor, CAR). CARs are fusion proteins formed by an antigen-binding region (Ag), generally a fragment of recombinant mAb with a single chain or scFV (single chain Fv), and by the cytoplasmic portion of a chain of the TCR / complex. CD3 of the T lymphocyte is necessary for the activation of effector cells. The presence of a single intracytoplasmic activation signal or the combination of two or three of them, defines the CARs as first, second and third generation respectively. Recently the co-expression of inducible IL-12 has led to the development of so-called fourth generation CARs. First-generation CARs have been ineffective, and very persistent *in vivo*, while second and third generation CARs are giving results with relevant clinical impact. In 2010, the FDA approved for the first time the use in clinical trials of genetically modified T lymphocytes, expressing CARs that specifically recognize CD19 or CD20, in patients with recurrent lymphomas. Since then, several clinical trials, using autologous and allogeneic T lymphocytes expressing, among others, anti-CD 19 CARs have shown a high degree of success in patients with different B-line hematological neoplasms. Part of these studies have led to the approval by the FDA in 2017 and by the EMA in 2018 of two drugs: Kymriah (Novartis) for the treatment of refractory or relapsed (r / r) acute lymphoblastic (ALL) leukemias and Lymphoma Diffuse Large Cell (LDCG) r / r and Yescarta (Kite) for the treatment of LDCG r / r including primary mediastinal lymphoma. From the pool of patients treated with anti-CD 19 CAR-T autologous lymphocytes in pivotal clinical trials and subsequently in the real-life setting, we have learned several lessons. From the pool of patients treated with anti-CD19 CAR-T autologous lymphocytes in pivotal clinical trials and subsequently in the real-life setting, we have learned several lessons. The first lesson is that the continuous expression of CAR causes acute toxicities not previously observed with other antineoplastic therapies, which are fundamentally two: cytokine release syndrome (CRS) and neurological toxicity known as encephalopathy syndrome or neurotoxicity syndrome associated immune effector cells (ICANS). In this sense, a strong relationship has been observed between the degree of severity of both toxicities and the maximum peak of expansion *in vivo* of CAR-T and with the levels of circulating cytokines in relation to the functionality of the CAR-T cells. The second lesson is that the treatment received by patients previously affects the availability and quality of T cells for the production of CAR-T cells (most patients have received several lines of chemotherapy and / or immunomodulatory agents before of apheresis to obtain lymphocytes for CAR production, which means that certain cases cannot be obtained in sufficient quantity or quality). Finally, a high tumor mass at the time of CAR-T cell treatment is associated with poorer outcomes. In ALL, a percentage greater than 5% of blasts in bone marrow has a clear impact on the duration of the response, and the size of the lymphadenopathy has a similar influence in the case of chronic lymphatic leukemia (CLL) and CLLD. The negative impact of the tumor mass on the results of CAR-T immunotherapy is not surprising since it is well known that it also negatively affects any of the antineoplastic therapy modalities including polychemotherapy itself. In this sense, there are multiple factors that determine the deleterious effects of high tumor mass on treatment results, one of them being the high concentration of EXO from tumor cells in the peripheral blood of treated patients.

On the other hand, EXOs are defined as small vesicles of 30 to 100 nm, loaded and secreted by all cell types in both cultures and physiological conditions. This secretion, as discussed above, is significantly increased in tumor cells. Under normal physiological conditions, EXOs play a fundamental role in intercellular communication by exchanging proteins, nucleic lipids and acids with target cells, while in pathological processes related to angiogenesis, inflammation and coagulation, this vesicle may contain nucleic acids in the form of microRNA and mRNA and even oncogenes. Regardless of the origin and function of EXOs, these are formed by a process in which membranes are invagined to form vesicles within the endosomal compartment, producing multivesicular bodies (MVB).

While the role of EXOs as diagnostic markers is more than evident their therapeutic use is much reduced, due, in one hand to the absence in their surface of specific ligands or antibodies, on the other hand to the low efficiency in the protocols of EXOs purification. Few strategies have been carried out to provide EXOs on their surface with specific molecules. In this regard, EXOs derived from HEK293T with hyaluronate (LipHA-hEVs) have been developed as a ligand of CD44 over expressed in breast cancer; the SSTR1 antibody that specifically recognizes pancreatic cancer, or the anti-CD47 antibody present in various malignant cells, has also been incorporated. Perhaps one of the most innovative applications in the field of nanotechnology and immunotherapy is the production of EXOs from CAR-T cells, which present CAR on their surface, which would allow a specific lysis of tumor cells without the need for CAR-T cells themselves. In this sense, a work recently published by Fu y col has shown that it is possible to produce EXOs from CAR-T and that these EXO-CART exhibit anti-tumor activity.

However, the authors themselves highlight two important limitations: the existence of surface HLA molecules of EXO-CARTs that may induce some form of rejection, and the difficulty of obtaining a sufficiently high number of EXO-CART in order to consider therapeutic use.

So, there is still an unmet medical need of finding reliable procedures to improve the production of EXO from CAR-T cells. The present invention is focused on solving this problem and an innovative method for improving EXO production from CAR-T cells is herein provided.

### DESCRIPTION OF THE INVENTION

### Brief description of the invention

As explained above, the present invention refers to a method for improving the production of EXO from CAR-T cells by modifying the expression of specific genes. The invention also refers to CAR-T cells, or EXO derived thereof, wherein the expression of specific genes have been modified, and to pharmaceutical compositions comprising said CART-cells or EXO derived thereof.

Particularly, the main objective of the present invention is the generation of an effective and safe platform, based on genomic editing and addition techniques, to produce large-scale therapeutic EXO from universal CAR-T cells. The EXO will be preferably used in an allogenic context and synergistically with universal CAR-T-CD19 (B2M-/-) previously developed by the requesting team, thus increasing the antitumor efficacy of CAR-T therapies in hematological B-stretch neoplasms paving the way to their use in other cancer processes.

Such as it is explained in the Examples shown below, the present invention demonstrates that modifying the expression pattern of specific CART-cell genes has a direct impact on EXO production. Specifically, according to the present invention, the production of EXO from CART-cells can be improved by means of the deletion or expression inhibition of at least a gene selected from: ISG15, HGS, VTA and/or CHMP4C, or any combination thereof, preferably in combination with the over-expression of at least a gene selected from the list comprising: CD9, CD63 and/ ALIX, or any combination thereof.

So, the first embodiment of the present invention refers to an *in vitro* method (hereinafter the "method of the invention") for the production of EXO from CAR-T cells which comprises: a) Deletion or expression inhibition of the gene ISG15 from the genome of the cells, and b) Placing CAR-T cells thus obtained according to the step a) in a suitable media for the production of EXO.

The second embodiment of the present invention refers to the *in vitro* use of ISG15 to produce EXO from CAR-T cells.

In a preferred embodiment, the step a) of the method of the invention further comprises the deletion or expression inhibition of a gene selected from the list comprising: HGS, VTA and/or CHMP4C.

In a preferred embodiment, the method of the invention further comprises the over-expression of at least a gene selected from the list comprising: CD9, CD63 and/ ALIX.

In a preferred embodiment, the over-expression of the genes CD9, CD63 and/or ALIX is carried out by transfecting cells with a lentiviral vector expressing the cDNA of CD9, CD63 and/or ALIX; and wherein the deletion of the genes ISG15, HGS, VTA and/or CHMP4C is carried out by using gene editing techniques, preferably CRISPR/Cas technology, Zinc Finger Nucleases or TALENs Gene Editing.

In a preferred embodiment, the CAR-T cells which produces EXO are CART cells targeting CD19 antigen.

The third embodiment of the present invention refers to CAR-T cells, or EXO derived thereof (hereinafter CAR-T cells or EXO of the invention), characterized by comprising a deletion or expression inhibition of the gene ISG15.

In a preferred embodiment, the CAR-T cells, or EXO derived thereof, further comprise a deletion or expression inhibition of a gene selected from the list comprising: HGS, VTA and/or CHMP4C.

In a preferred embodiment, the CAR-T cells, or EXO derived thereof, are further characterized by over-expressing at least a gene selected from the list comprising: CD9, CD63 and/ ALIX.

The fourth embodiment of the present invention refers to a pharmaceutical composition (hereinafter pharmaceutical composition of the invention) comprising the CAR-T cells or EXO of the invention and, optionally, pharmaceutically acceptable excipients or carriers.

The fifth embodiment of the present invention refer to the pharmaceutical composition of the invention for use as medicament, preferably in the treatment of B-cell malignancies, most preferably CD19+ malignancies.

Alternatively, the present invention also refers to a method for treating a patient, preferably a patient suffering from B-cell malignancies, most preferably CD19+ malignancies, which comprises the administration of the therapeutically effective amount of the pharmaceutical composition of the invention.

For the purpose of the present invention the following terms are defined:
- The term "comprising" means including, but not limited to, whatever follows the word "comprising". Thus, use of the term "comprising" indicates that the listed elements are required or mandatory, but that other elements are optional and may or may not be present.
- The term "consisting of' means including, and limited to, whatever follows the phrase "consisting of'. Thus, the phrase "consisting of' indicates that the listed elements are required or mandatory, and that no other elements may be present.
- "Pharmaceutically acceptable excipient or carrier" refers to an excipient that may optionally be included in the compositions of the invention and that causes no significant adverse toxicological effects to the patient.
- By "therapeutically effective dose or amount" of the composition of the invention is intended an amount that, when administered to the patient, brings about a positive therapeutic response. The exact amount required will vary from subject to subject, depending on the species, age, and general condition of the subject, the severity of the condition being treated, mode of administration, and the like. An appropriate "effective" amount in any individual case may be determined by one of ordinary skill in the art using routine experimentation, based upon the information provided herein and the common general knowledge.

### Description of the figures

**Figure 1****.** Indirect staining of CAR in the surface of the EXO extracted from CAR-T cells.
**Figure 2****.** SEM based structural characterization of EXO obtained from WT and ISG KO cells.
Interestingly, although SEM is not a quantitative technique, we found a greater number of exosomes per field in ISG-15 KO purified particles.
**Figure 3****.** Transmission electron microscopy (TEM) image of exosome extracted from WT and ISG 15 cells. This figure confirms the expected size range of purified exosomes and the morphological integrity of purified exosomes either in treated cells (ISG ko) and in the WT cells.
**Figure 4****.** The atomic force microscopy (AFM) was used in order to characterize the isolated EXO. AFM based structural characterization of single EXO obtained from WT and ISG KO cells. A higher size and number of EXO is observed in the case of cells comprising ISG15 deletion, which is an indication of an improved production of EXO.
**Figure 5****.** Enumeration and sizing of EXO derived from WT and ISG15 KO Jurkat cells using NanoSight NS300. According to this analysis, there is a steady increase in EXO concentration in the supernatant obtained for ISG15 KO cells, in comparison with the wild type.

### Detailed description of the invention

The present invention is illustrated by means of the Examples set below, without the intention of limiting its scope of protection.

### Example 1. Obtention of HLA KO

For the generation of HLA KO cells, the B2M gene has been chosen since the B2M protein forms a heterodimer with Class I HLA proteins. The absence of the B2M protein makes it impossible to present HLA molecules on the surface. the Chop chop platform (https://chopchop.cbu.uib.no) has been used for the design of the B2M locus guide, on which we were able to obtain the sequences of two RNAs guides (see **Table 1**).

**Table 1**

| **GUIDE SEQUENCE** | **EXON** | **CUT SITE** | **PAM** | **ON TARGET SCORE** | **OFF TARGETs** |
|---|---|---|---|---|---|
| SEQ ID NO: 1 AAGTCAACTTCAATGTCGGA | 1 | 44,715,515 | GGG | 0,582 | 0,0,0,4,71 |
| SEQ ID NO: 2 ACTCACGCTGGATAGCCTCC | 1 | 44,711,603 | CGG | 0,584 | 0,0,0,2,52 |

Once the sequence of the 2 guides has been obtained, T B2M-/- cells have been generated, and the expression of B2M and HLA on the surface has been analyzed. After analyzing the editing efficiency of the two guides, the best of the guides for further analysis has been selected.

The gene editing efficiency of B2M locus, and time course expression over the time of B2M in the surface of the edited cells were assayed. At days 12 almost all the treated cells are negative for B2M expression, therefore they are HLA negative.

### Example 2. Obtention of B2M-/--CART

### Example 2.1. Generation of 3G-CAR lentiviral vector

For the obtention of lentiviral vectors expressing CAR-3G (third generation) the optimized method of the laboratory has been followed, consisting of a triple transfection of HEK-293T packing cells with lenti-EF1-CD19-3rd-CAR-eGFt plasmid (Creative Biolabs), pCMVDR8.91 (http://www.addgene.org/Didier_Trono) packaging plasmid and wrapped plasmid (VSV-G) (http://www.addgene.org/Didier_Trono). The transfection has been carried out by lipod (Signagen) or PEI reagent (Quimigen-23966-1). The cell supernatant containing viral particles was collected, filtered and frozen for further analysis and/or use for the production of third generation CAR-Ts.

### Example 2.2. Generation of 3G-CART

For the generation of third generation CAR-T, healthy donor T cells have been used, activated and transduced with a multiplicity of infection (MOI) of 10, i.e. 10 viral particles/cell, by a spinoculation (800 x g for 60 min at 32oC). The estimation of transduction efficiency, as in the previous case, has been estimated by both cytometry and PCR in real time.

The level of expression of CAR T CAR expression in the surface of the EXO was measured with a biotin-conjugated goat anti-murine Fab SP-longer Spacer IgG (Jackson Immunoresearch) and APC-conjugated streptavidin (ThermoFisher).

CART cells were incubated with target cells (CD19), for 48h and the lysis was monitored by the elimination of the GFP +/+ cells. The level of cell cytotoxicity was similar in both KO and WT CAR-T cell.

### Example 3. Obtention of EXO-CAR-T from CAR-T cells

The first EXO-CART purification was carried out using 40 ml of CAR-T supernatant and WT was processed and in order to purify endovesicles and EXO. We isolated EXO from the culture supernatant from CAR-T following the ultracentrifugation protocol. Based on their unique size and density. Briefly cells were cultured in medium TEXmax. Supernatant fractions were collected from overnight cell cultures and EXO were obtained by serial centrifugation as follow. Cells were pelleted (320g for 10 min) and the supernatant was centrifuged at 2000g for 15 min, to discards dead cells. The supernatant was collected and ultra-centrifugated at 10.000g for 30 min, finally the EXO was obtained by a centrifugation at 100.000g for 70 min.

### Example 3.1. Phenotypic EXO characterization

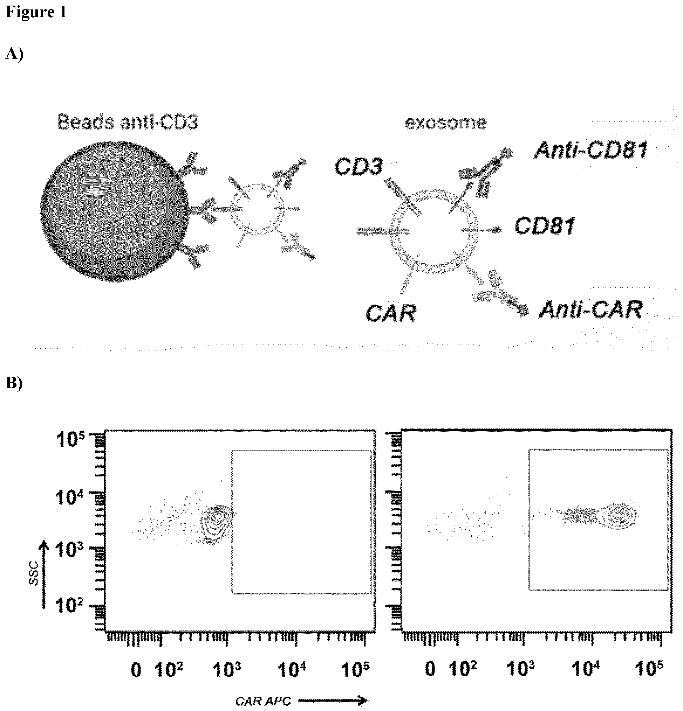

CAR expression in the surface of the EXO was measured with a biotin-conjugated goat anti-murine Fab SP-longer Spacer IgG (Jackson Immunoresearch) and APC-conjugated streptavidin (ThermoFisher). Detection was achieved thought the conjugation of Exo-CART with the magnetic beads-antiCD3. **Figure 1** shows the indirect staining of CAR in the surface of the EXO extracted from CAR-T cells.

### Example 3.2. Functional characterization of EXO-CAR-T

In order to check the lytic capacity of the EXO, we performed a cytolytic assay by the incubation of EXO with CD19 positive cells. For this purpose, target cells (CD19+), Namalwa and Nalm-6 cells expressing CD19 were co-cultured in duplicate in 96-well U-bottom plates, and incubated with EXO-CAR-T cells at various EXO-CAR-target (E:T) ratios (5:1, 10:1, 50:1) in non-supplemented TexMACs during 48h as indicated. Percentage of lysis was determined by flow cytometry related to basal lysis produced by non-transduced T cells. Specific lysis was determined as follows: 1-(%CD19/% HL-60 in CAR-T cells condition/ %CD19+/%HL-60 in NTD condition) x100.

### Example 3.3. Optimization of the EXO secretion or production

We aim to improve the production of EXO from T cells (EXO-T), by elimination of ISG15 and HGS and/or VPS4 genes and/or by over-expression of CD9, CD63 and ALIX genes, genomic addition and editing techniques will be used, and the best combinations of overexpressed and KO genes will be selected for the production of EXO. Due to the difficulty to work with T cell, all the preliminary experiments were performed using Jurkat cell (an immortalized line of human T lymphocyte cells).

### Example 3.3.1. Deletion or expression inhibition of the gene ISG15

ISG15 is a 15 kDa protein that belongs to the family of ubiquitin-like modifiers (UBLs) with a broad action and involvement in several physiological pathway. Our aim in this way is to eliminate the expression of ISG15 and the KO experiment were performed by the design of gRNA according to https://chochop.cbu.uib.no. Particularly, the KO experiment was carried out by forming an RNP using CAS9 HF proteins and gRNA. After gene editing (GE), a PCR reaction was carried out to analysis the GE efficiency:

**Table 2**

| Gene ISG15 | | | | | |
|---|---|---|---|---|---|
| gRNA sequence | SEQ ID NO: 3 GGTACGGCTGACGCAGACCG | | | TM | amplicon |
| | | | | | |
| Primer Fw 1 | SEQ ID NO: 4 GAGCATCCTGGTGAGGAATAAC | | | 60.0 | 213 |
| Primer Rev1 | SEQ ID NO: 5 CGCAGATTCATGAACACGGT | | | 62.1 | |

The PCR product was submitted to an interference of CRISPR Edits to analysis knockout and Knock-in level using the httpps://www.synthego.com.

We isolated EXO from the culture supernatant from Jurkat cells subpopulations following the ultracentrifugation protocol. Based on their unique size and density, EXO and extracellular vesicles purification will be confirmed by TEM, AFM, Western blot, NanoSight and SEM.

### SEM analysis

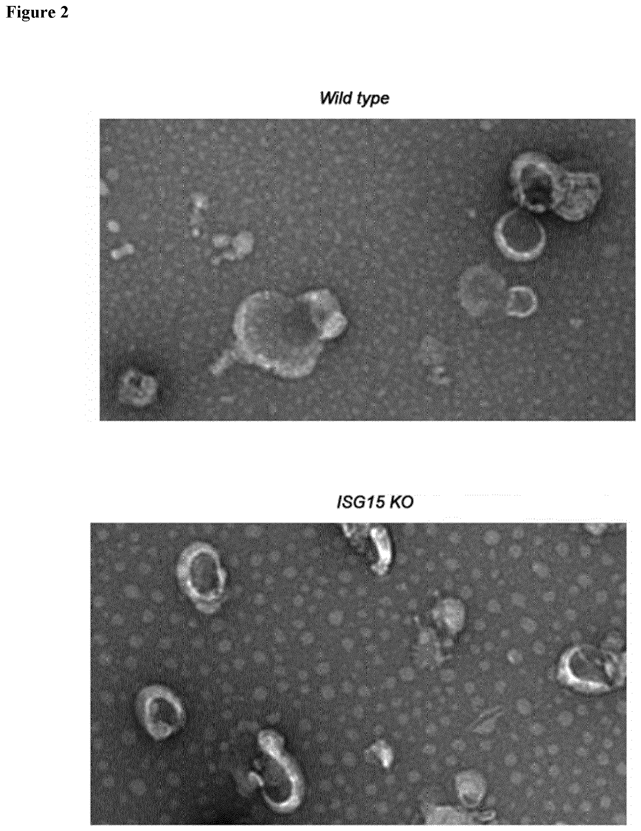

Under SEM analysis, EXO isolated from WT and ISG15KO cells exhibit a round morphology and uniform, unimodal distribution in size, even so the amount of EXO obtained for ISGKO cells looks higher that those obtained from WT (as observed in the preliminary data of AFM analysis) (see **Figure 2**).

### TEM analysis

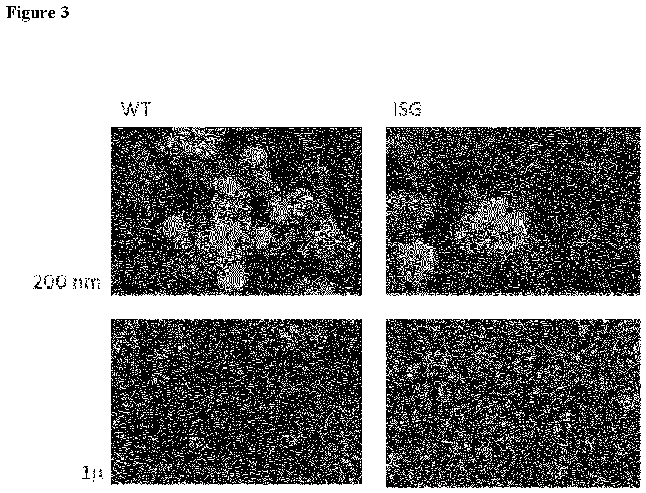

As shown in **Figure 3****,** a greater number of exosomes were found per field in ISG-15 purified particles.

### AFM analysis

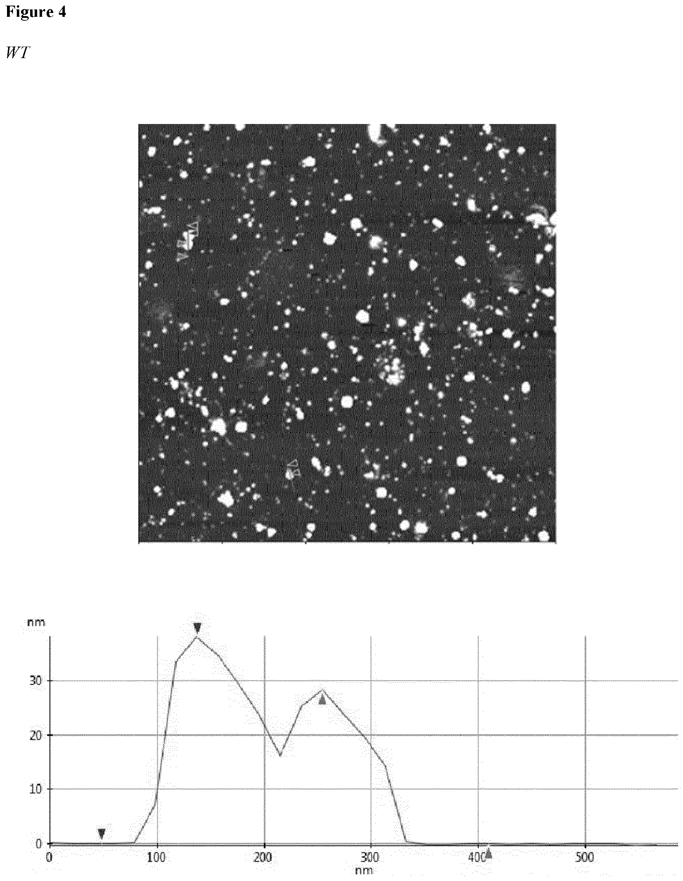

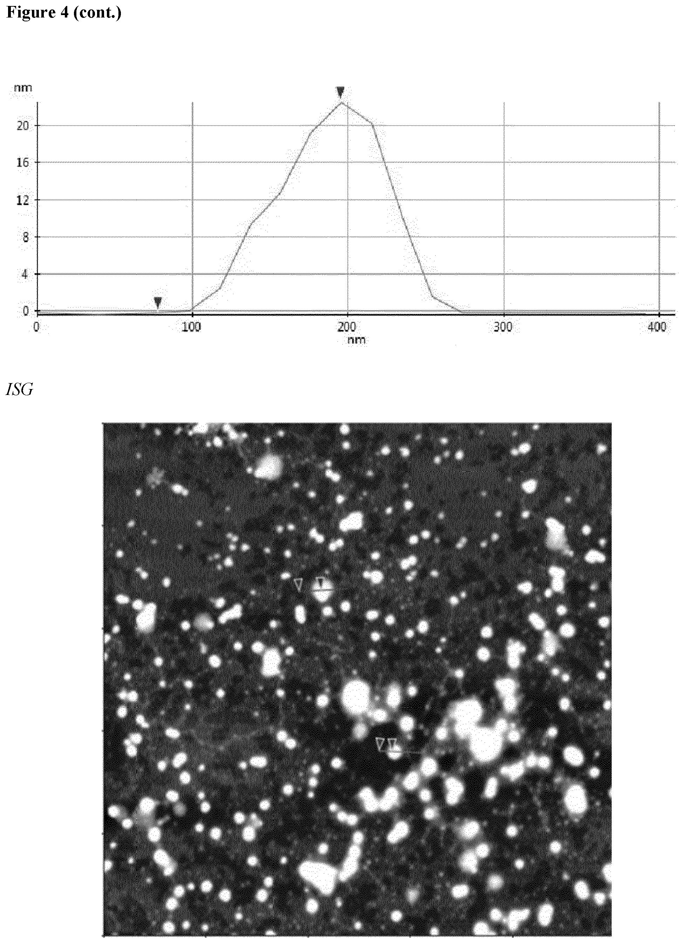

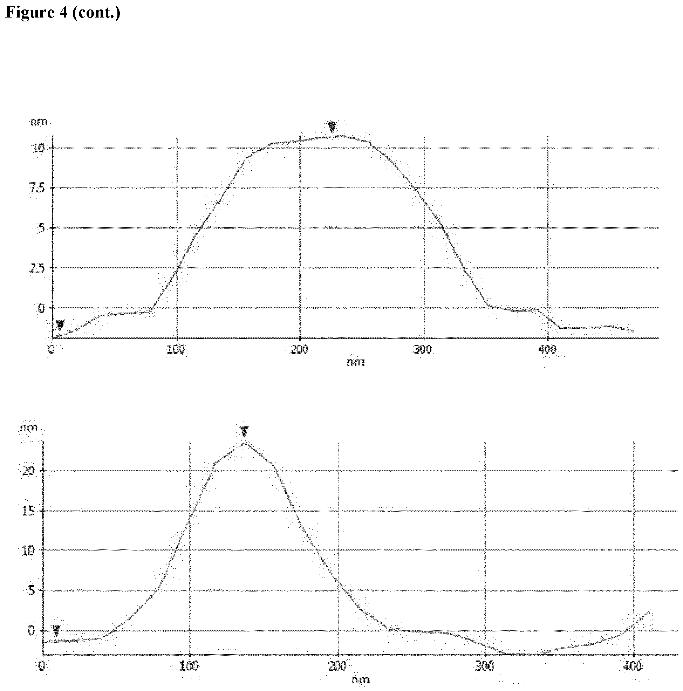

The atomic force microscopy (AFM) was used in order to characterize the isolated EXO obtained from WT and ISG KO cells. A higher size and number of EXO is observed in the case of cells comprising ISG15 deletion, which is an indication of an improved production of EXO (see **Figure 4**)**.**

### NanoSight analysis

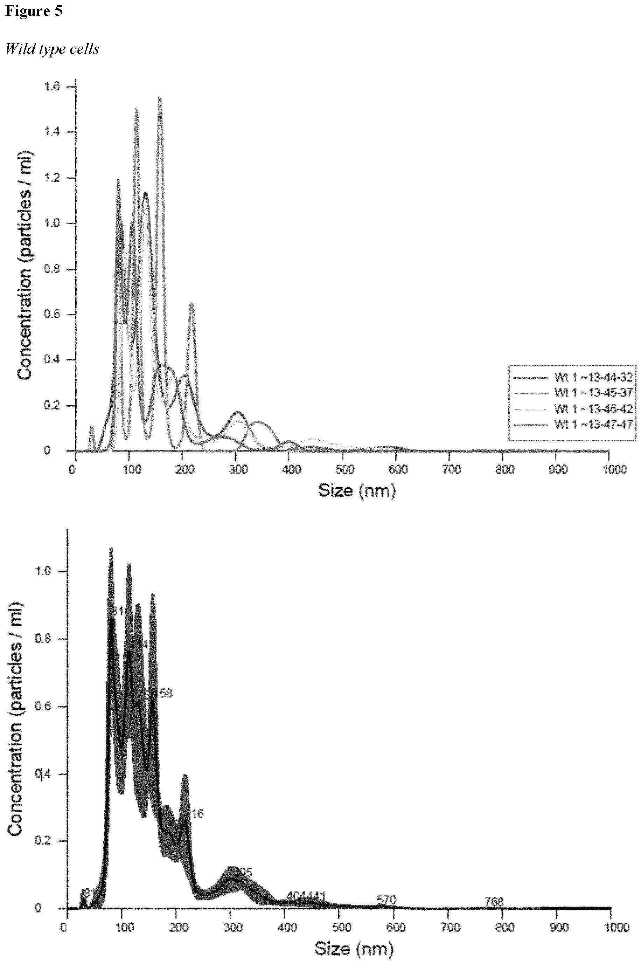

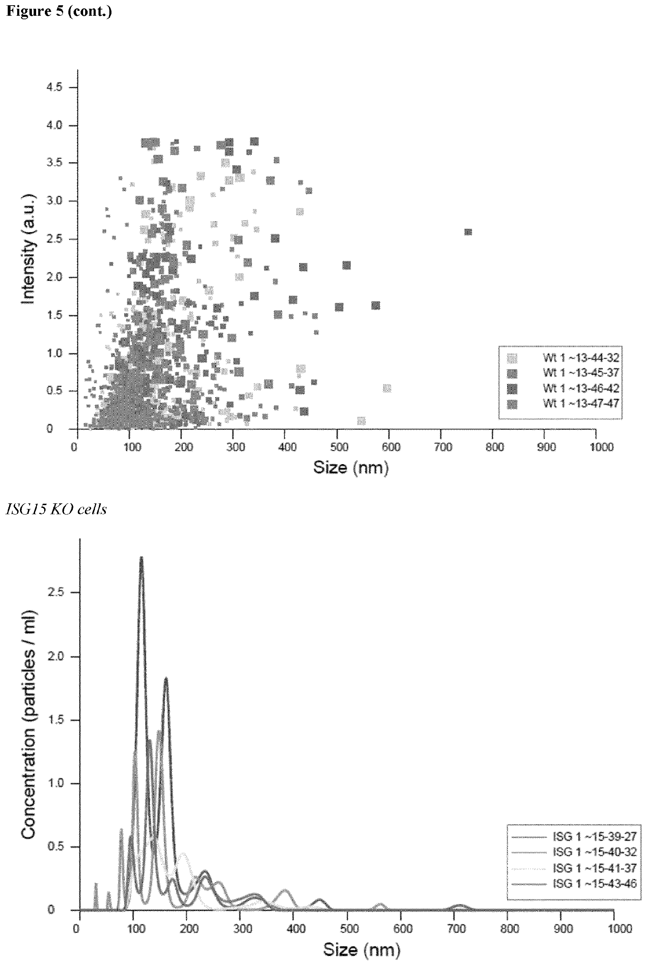

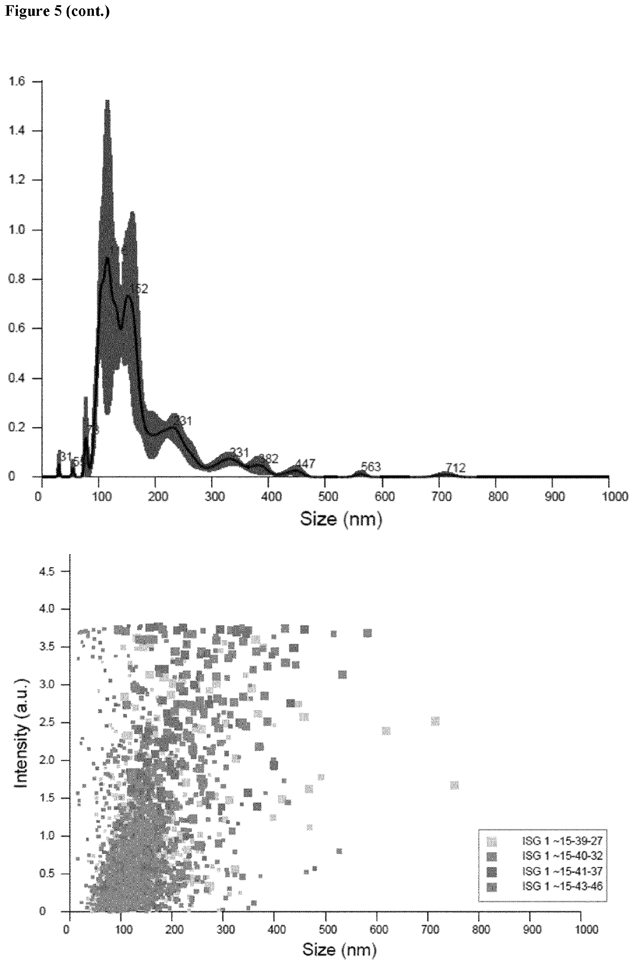

According to this analysis, such as it can be observed in **Figure 5****,**

## Claims

1. *In vitro* method for the production of exosomes from CAR-T cells which comprises:
a. Deletion or expression inhibition of the gene ISG15 from the genome of the cells, and
b. Placing CAR-T cells thus obtained according to the step a) in a suitable media for the production of exosomes.

2. *In vitro* method, according to claim 1, wherein the step a) further comprises the deletion or expression inhibition of a gene selected from the list comprising: HGS, VTA and/or CHMP4C.

3. *In vitro* method, according to any of the claims 1 or 2, which further comprises the over-expression of at least a gene selected from the list comprising: CD9, CD63 and/ ALIX.

4. *In vitro* method, according to any of the previous claims, wherein the over-expression of the genes CD9, CD63 and/or ALIX is carried out by transfecting cells with a lentiviral vector expressing the cDNA of CD9, CD63 and/or ALIX; and wherein the deletion of the genes ISG15, HGS, VTA and/or CHMP4C is carried out by using gene editing techniques, preferably CRISPR/Cas technology, Zinc Finger Nucleases or TALENs Gene Editing.

5. *In vitro* method, according to any of the previous claims, wherein the CAR-T cells are CART cells targeting CD19 antigen.

6. *In vitro* use of ISG15 for the production of exosomes from CAR-T cells.

7. *In vitro* use of ISG15, according to claim 6, in combination with HGS, VTA and/or CHMP4C for the production of exosomes from CAR-T cells.

8. CAR-T cells, or exosomes derived thereof, **characterized by** comprising a deletion or expression inhibition of the gene ISG15.

9. CAR-T cells, or exosomes derived thereof, according to claim 8, **characterized by** further comprising a deletion or expression inhibition of a gene selected from the list comprising: HGS, VTA and/or CHMP4C.

10. CART-cells, or exosomes derived thereof, according to any of the claims 8 or 9, further **characterized by** over-expressing at least a gene selected from the list comprising: CD9, CD63 and/ ALIX.

11. Pharmaceutical composition comprising the CAR-T cells or exosomes derived thereof of any of the claims 8 to 10 and, optionally, pharmaceutically acceptable excipients or carriers.

12. Pharmaceutical composition, according to claim 11, for use as medicament.

13. Pharmaceutical composition, according to claim 12, for use in the treatment of B-cell malignancies, preferably CD19+ malignancies.
